# EUROPÄISCHE PATENTSCHRIFT

(11) **EP 1 043 038 B1**
(45) Veröffentlichungstag und Bekanntmachung des Hinweises auf die Patenterteilung: **05.12.2001**
(21) Anmeldenummer: 00104245.6
(22) Anmeldetag: 02.03.2000
(51) Int. Cl.: A61M 5/14

(54) **Kombinationssystem von mehreren medizinischen Geräten zur vollständig intravenösen Anästhesie**
System comprising several medical devices for complete intravenous anaesthesia
Système comprenant plusieurs dispositifs médicaux pour anaesthésie intraveineuse complète

(30) Priorität: 03.04.1999 DE 19915299
(43) Veröffentlichungstag der Anmeldung: 11.10.2000
(73) Patentinhaber: Codan Holding GmbH, 23738 Lensahn (DE)
(72) Erfinder: Knorr, Olaf, 23562 Lübeck (DE)
(74) Vertreter: Fleck, Thomas, Dr. Dipl.-Chem.

(56) Entgegenhaltungen:
- EP-A- 0 268 700
- EP-A- 0 653 217
- US-A- 4 447 230
- US-A- 5 074 334

## Beschreibung

Die vorliegende Erfindung betrifft ein Kombinationssystem nach dem Oberbegriff des Anspruches 1.

Ein derartiges Kombinationssystem ist im Stand der Technik nur aus Einzelkomponenten bekannt.

Bei der zur Zeit üblichen Arbeitsweise für die Anwendung einer total intravenösen Anästhesie werden mindestens zwei Dreiwegehähne, sowie zwei Spritzenpumpen-Verlängerungen benötigt. Beide Dreiwegehähne werden miteinander konnektiert. An die Dreiwegehähne werden jeweils die Spritzenpumpen-Verlängerungen konnektiert. Am distalen (Patienten nahen) Mehrweghahn wird die Patientenzugangsleitung konnektiert. Nachdem diese Arbeitsschritte vollzogen wurden, muß das gesamte "Set" entlüftet werden und ist dann dementsprechend einsatzbereit. Alternativ wird am proximalen Mehrweghahn ein Infusionsgerät für Trägerlösungen konnektiert. Diese Arbeitsschritte sind nach jeder erfolgten Operation notwendig, da diese sod zusammengebastelten "Systeme" nur für den einmaligen Gebrauch zugelassen sind.

Der Erfindung liegt deshalb die Aufgabe zugrunde, das eingangs genannte Kombinationssystem von mehreren medizinischen Geräten zur total intravenösen Anästhesie derart zu verbessern, daß es mehrfach bei unterschiedlichen Patienten angewendet werden kann, ohne daß häufige Entlüftungen und. Konnektionen, die z.B. eine Kontaminationsgefahr, Verwechslungsgefahr und einen Zeitaufwand in sich bergen, vorgenommen werden müssen.

Diese Aufgabe wird durch das im Anspruch 1 gekennzeichnete Kombinationssystem gelöst.

Die mit den Bezugszeichen 1, 2 und 4 gekennzeichneten medizinischen Geräte sind erfindungsgemäß werkseitig montiert und fest miteinander verbunden (z.B. verklebt). Diese Kombination bildet ein geschlossenes System, welches Kontamination und Manipulation ausschließt. Ein mehrfacher Gebrauch des erfindungsgemäßen Kombinationssystems ist nur möglich, sofern nur die Patientenzugangsleitung von Operation zu Operation durcheine neue, sterile ersetzt wird. Letztere bildet eigentlich den Teil des erfindungsgemäßen Kombinationssystems, welches den mehrfachen Gebrauch ermöglicht und somit viel wirtschaftlicher ist als das vorbekannte System, welches aus einzelnen Komponenten besteht, die vor jeder Operation zusammengebaut werden müssen und die o.a Nachteile beinhaltet. Auch hier bringt das erfindungsgemäße Kombinationssystem eine besondere Arbeitserleichterung mit sich und ist gleichzeitig umweltfreundlicher durch die entsprechende Müllvermeidung.

Weitere Vorteile und Merkmale gehen aus den Unteransprüchen hervor, die gemeinsam mit dem Hauptanspruch auch von erfinderischer Bedeutung sein können.

Im folgenden werden zwei Ausführungsbeispiele der vorliegenden Erfindung zum besseren Verständnis derselben anhand der Zeichnungen näher erläutert.

Es zeigt:
- Fig. 1: eine schematische Seitenansicht des erfindungsgemäßen Kombinationssystems nach einer ersten Ausführung; und
- Fig. 2: eine schematische Ansicht eines erfindungsgemäßen Kombinationssystems nach einer zweiten Ausführung; und

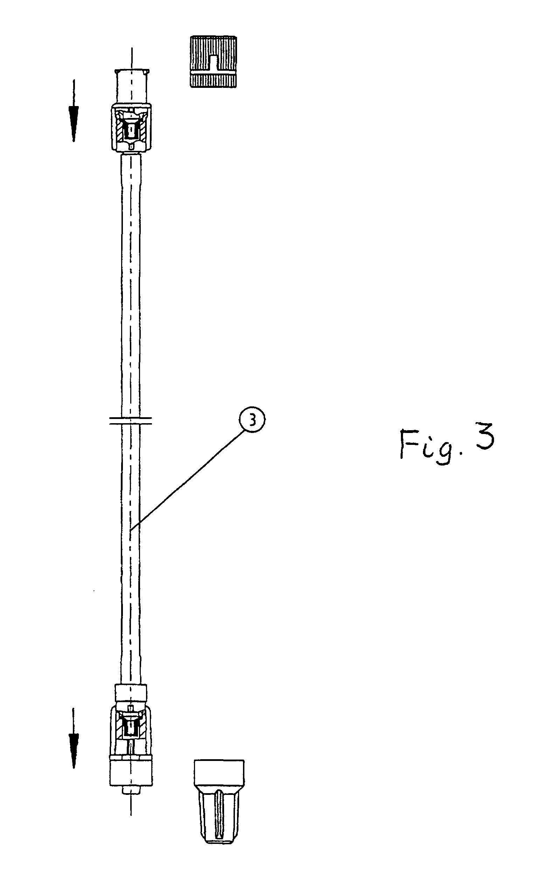

- Fig. 3: eine schematische Seitenansicht der erfindungsgemäßen Patientenzugangsleitung als Einzelheit.

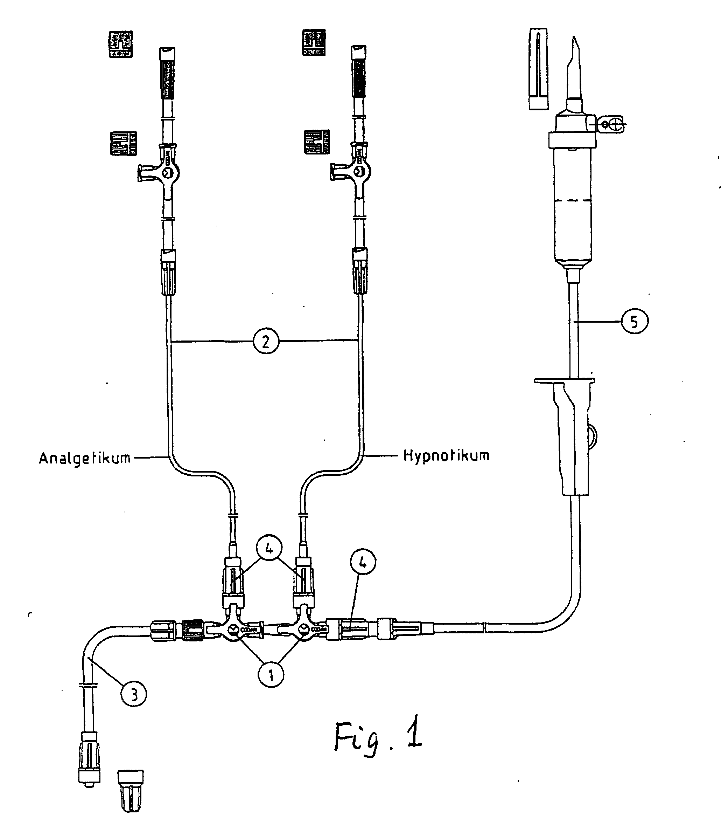

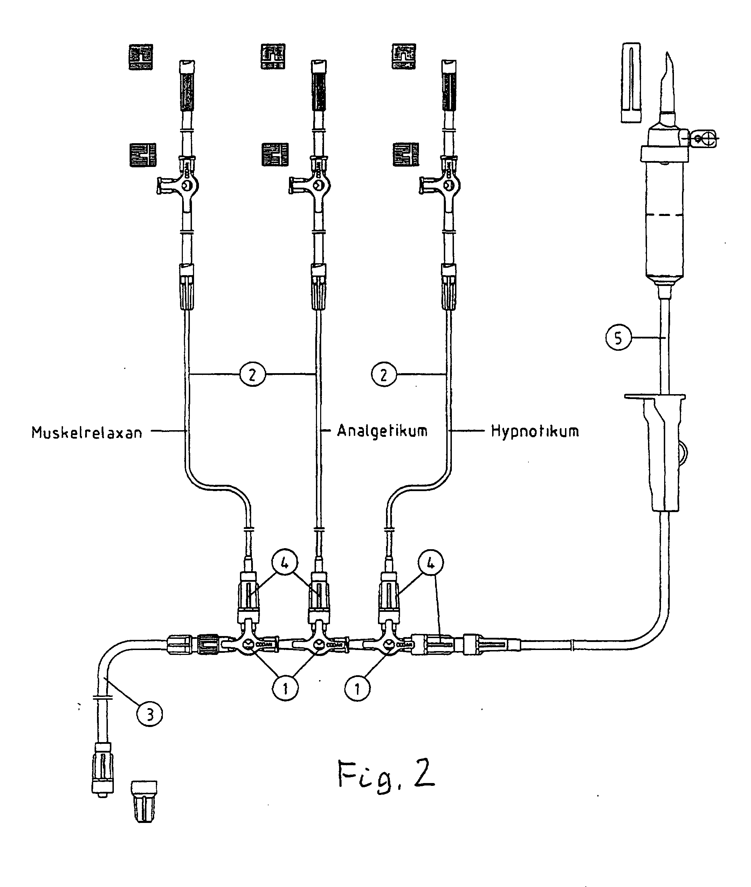

In Fig. 1 ist das erfindungsgemäße Kombinationssystem mit 10 bezeichnet, während es in Fig. 2 allgemein mit 20 bezeichnet ist. Es stellt ein in sich geschlossenes System dar, bei dem sämtliche Verbindungen unlösbar miteinander verbunden sind. Die Ausnahmen bilden hier die Patientenzugangsleitung 3 und das Infusionsgerät 5, mit ihren entgegen der Flußrichtung liegenden Rückflußbarrieren.

Das erfindungsgemäße Kombinationssystem, das auch als CODAN- TIVA-Set 2 bzw. 3 bezeichnet wird, setzt sich aus zwei, drei oder noch weiteren Spritzenpumpenleitungen und/oder Übertragungsleitungen 2 zusammen, die endständig zu den integrierten Mehrweghähnen/Verbindern oder Hahnbank 1 mit Rückflußbarrieren 4 ausgestattet sind. Die Rückflußbarrieren 4 vor den Spritzenpumpen-Übertragungsleitungen 2 und dem Infusionsgerät. 5, beugen retrograden Vermischungen vor, die nachweislich immer wieder bei solchen Parallelinfusionen entstehen. Am Patienten entfernten Ende der Spritzenpumpen- bzw. Übertragungsleitung 2 können auf Wunsch jeweils noch Mehrweghähne/Verbinder mit oder ohne Schlauchansatz integriert werden. Die Mehrweghähne/Verbinder dienen zum wiederholten Befüllen des angeschlossenen Verabreichungssystems, um ein in sich geschlossenes System zu gewährleisten. Die Schlauchansätze würden das Anschließen an eine Spritzenpumpenspritze und das Einlegen in die verschiedenen Spritzenpumpen erleichtern. Sämtliche Verbindungen oder Konnektoren sind unlösbar miteinander verbunden und bilden somit das geschlossene erfindungsgemäße System 10 und 20, um Kontamination und Manipulation auszuschließen.

Am distalen Ende der Systeme 10 und 20 befindet sich entkonnektierbar eine Patientenzugangsleitung 3. Diese ist beidseitig entgegen der Flußrichtung mit Rückflußbarrieren ausgestattet. Die Rückflußbarrieren verhindern in Zusammenhang mit der Gestaltung der Patientenzugangsleitung eine Reflux-Kontamination des geschlossenen Systems von der Patientenseite her. Nur durch diese Patientenzugangsleitung 3 ist die Mehrfachverwendung möglich. Nach der Operation wird die Patientenzugangsleitung 3 entkonnektiert und entsorgt, und durch eine sterile, neue ersetzt. Das erfindungsgemäße Kombinationssystem und die darin befindlichen Medikamente sind für den Einsatz von mehreren Patienten bzw. Operationen weiter anwendbar. Diese Mehrfachanwendung für dieses Anwendungsgebiet ist neu und erfinderisch.

Das am proximalen Ende entkonnektierbare Infusionsgerät 5 dient zur Verabreichung von Trägerlösungen. Dieses wird optional angeboten. Es muß entkonnektierbar sein, damit der Anwender nach versehentlichem Leerlaufen des Infusionsgerätes nur dieses austauschen muß, und nicht das komplette Set. Es wird ebenfalls durch eine Rückflußbarriere 4 abgesichert, um Vermischungen zu vermeiden.

Die Erstausstattung wird werksseitig anwenderfertig ausgeliefert. Für Folgeoperationen wird die Patientenzugangsleitung 3 gegen eine neue, sterile ausgewechselt. Damit ist das System wieder einsatzbereit, ohne das gesamte System neu vorzubereiten, wie z.B. entlüften.

Als weitere Variante kann ein Infusionsfilter vorzugsweise zwischen Patientenzugangsleitung 3 und Mehrweghähne/Verbinder 1 konnektiert werden.

## Patentansprüche

1. Kombinationssystem von mehreren medizinischen Geräten zur total intravenösen Anästhesie (TIVA), umfassend mindestens zwei miteinander direkt verbindbare Mehrweghähne/Verbinder (1), mindestens zwei daran anschließbare Spritzenpumpen- und/oder Übertragungsleitungen (2) und eine mit einem der Mehrweghähne/Verbinder verbindbare Patientenzugangsleitung (3), sowie gegenüberliegend verbunden mit einem Infusionsgerät (5),
**dadurch gekennzeichnet, daß** sämtliche Geräte, abgesehen von der Patientenzugangsleitung (3) und dem Infusionsgerät (5), unlösbar unter Ausbildung eines geschlossenen Systems werksseitig miteinander verbunden sind, wobei die Spritzenpumpen- und/oder Übertragungsleitungen (2) distal, sowie der proximale Mehrweghahn/Verbinder (1) an ihrem Ende jeweils eine Rückflußbarriere (4) an dem ihm zugeordneten Mehrweghahn/Verbinder (1) haben.

2. Kombinationssystem nach Anspruch 1, **dadurch gekennzeichnet, daß** die Patientenzugangsleitung (3) an ihren beiden Enden mit Rückflußbarrieren (4) versehen ist, die lediglich eine Strömungsrichtung hin zum Patienten gestatten und nur somit die Mehrfachverwendung zuläßt.

3. Kombinationssystem nach Anspruch 1 oder 2, **dadurch gekennzeichnet, daß** die umlösbere Verbindung der medizinischen Geräte (1,2,4) durch ein Kunststoffklebemittel oder ein Schweißverfahren hergestellt ist.

4. Kombinationssystem nach einem der Ansprüche 1 bis 3, **dadurch gekennzeichnet, daß** die Rückflußbarrieren (4) Rückschlagventile sind.

5. Kombinationssystem nach einem der Ansprüche 1 bis 4, **dadurch gekennzeichnet, daß** zwei bzw. drei miteinander verbundene Mehrweghähne/Verbinder oder eine Hahnbank (1) enthalten sind.

6. Kombinationssystem nach einem der Ansprüche 1 bis 5, **dadurch gekennzeichnet, daß** zwischen Patientenzugangsleitung (3) und den Mehrweghähnen/Verbindern bzw. Hahnbank (1) ein Infusionsfilter zwischengeschaltet ist.

7. Kombinationssystem nach einem der Ansprüche 1 bis 6, **dadurch gekennzeichnet, daß** ein Infusionsgerät konnektiert für Trägerlösungen enthalten ist.

## Claims

1. Combination system of several medical instruments for complete intravenous anesthesia (TIVA), comprising at least two directly interconnectable multiway taps/connectors (1), at least two syringe pump and/or transfer lines (2) connectable thereto and a patient access line (3) connectable to the multiway taps/connectors and facing said line and connected thereto an infusion apparatus (5), **characterized in that** all the instruments, apart from the patient access line (3) and infusion apparatus (5) are permanently interconnected in the factory so as to form a closed, integrated system, the syringe pump and/or transfer lines (2) having on the distal side and the proximal multiway tap/connector (1) at its end in each case a return flow barrier (4) on the multiway tap/connector (1) associated therewith.

2. Combination system according to claim 1, **characterized in that** the patient access line (3) is provided at both ends with return flow barriers (4), which only allow one flow direction to the patient and this forms the basis for multiple use.

3. Combination system according to claim 1 or 2, **characterized in that** the permanent connection of the medical instruments (1, 2, 4) is provided by a plastic adhesive or a welding process.

4. Combination system according to one of the claims 1 to 3, **characterized in that** the return flow barriers (4) are check valves.

5. Combination system according to one of the claims 1 to 4, **characterized in that** two or three interconnected multiway taps/connectors or a tap bank (1) are provided.

6. Combination system according to any one of the claims 1 to 5, **characterized in that** an infusion filter is interposed between the patient access line (3) and multiway taps/connectors or tap bank (1).

7. Combination system according to any one of the claims 1 to 6, **characterized in that** a connected infusion apparatus is provided for carrier solutions.

## Revendications

1. Système combiné de plusieurs appareils médicaux pour anesthésie endoveineuse générale, comprenant au moins deux raccords à clapet sélecteur (1), susceptibles d'être raccordés directement l'un avec l'autre, au moins deux conduites pour pompe à injection et/ou conduites d'acheminement (2), qui peuvent être connectées auxdits raccords, et une conduite d'admission vers le patient (3) qui peut être raccordée à l'un des raccords à clapet sélecteur, de même que relié avec un dispositif de perfusion (5) disposé en face, **caractérisé en ce que** tous les appareils, à l'exception de la conduite d'admission vers le patient (3) et du dispositif de perfusion (5), sont assemblés les uns aux autres de manière inamovible en usine, en formant un système fermé, des barrières de reflux (4) étant prévues sur l'extrémité distale de chacune des conduites pour pompe à injection et/ou conduites d'acheminement (2), de même que sur le raccord à clapet sélecteur (1) proximal, sur l'extrémité du raccord à clapet sélecteur (1) qui lui est associée.

2. Système combiné selon la revendication 1, **caractérisé en ce que** la conduite d'admission vers le patient (3) est munie sur ses deux extrémités de barrières de reflux (4), qui autorisent uniquement un sens d'écoulement vers le patient et permettent, seulement de ce fait, un usage multiple du système.

3. Système combiné selon la revendication 1 ou 2, **caractérisé en ce que** l'assemblage inamovible des appareils médicaux (1, 2, 4) est réalisé au moyen d'une colle pour matière plastique ou par un procédé de soudage.

4. Système combiné selon l'une des revendications 1 à 3, **caractérisé en ce que** les barrières de reflux (4) sont des clapets anti-retour.

5. Système combiné selon l'une des revendications 1 à 4, **caractérisé en ce que** ledit système comporte deux ou trois raccords à clapet sélecteur reliés l'un à l'autre ou une ligne de clapets (1).

6. Système combiné selon l'une des revendications 1 à 5, **caractérisé en ce qu'**un filtre de perfusion est monté entre la conduite d'admission vers le patient (3) et les raccords à clapet sélecteur ou la ligne de clapets (1).

7. Système combiné selon l'une des revendications 1 à 6, **caractérisé en ce que** ledit système comporte un dispositif de perfusion raccordé, destiné à administrer des solutions porteuses.
